(19) Europäisches Patentamt
European Patent Office
Office européen des brevets

(11) Publication number: **0 015 628**
**B1**

(12) **EUROPEAN PATENT SPECIFICATION**

(45) Date of publication of patent specification: **30.01.85**

(51) Int. Cl.[4]: **A 01 N 43/40**, C 07 D 213/74

(21) Application number: **80200212.1**

(22) Date of filing: **06.03.80**

# (54) Use as fungicides of pyridyliminomethylbenzene derivatives and acid-addition salts thereof; pyridyliminomethylbenzene derivatives and their acid-addition salts; a process for preparing such compounds and intermediates for use in such process; and fungicidal compositions comprising such compounds.

(30) Priority: **13.03.79 GB 7908822**

(43) Date of publication of application:
**17.09.80 Bulletin 80/19**

(45) Publication of the grant of the patent:
**30.01.85 Bulletin 85/05**

(84) Designated Contracting States:
**AT BE CH DE FR GB IT NL SE**

(56) References cited:
**EP-A-0 000 816**
**DE-A-2 756 771**
**FR-A-1 437 425**
**NL-C- 100 994**
**US-A-2 472 292**
**US-A-3 472 743**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Ten Haken, Pieter**
**Konkelboet The Street Eastling**
**Near Faversham Kent (GB)**
Inventor: **Webb, Shirley Beatrice**
**17 North Street Ashford Road**
**Sheldwich Near Faversham Kent (GB)**

(74) Representative: **Hunter, Keith Roger Ian et al**
**4 York Road**
**London SE1 7NA (GB)**

**EP 0 015 628 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the use of pyridyliminomethylbenzene derivatives and acid-addition salts thereof as fungicides, and to certain such compounds per se.

There is a continuing need to find compounds having good fungicidal activity in a search for new potential fungicides. French patent Application 1437425 describes a small class of 3,5-dihalosalicylaldehyde-N-(heterocyclic)imines as having activity against fungi. However the compounds of the present invention show a greater fungicidal effect than the pyridyl derivatives of the forementioned specification. A further reference, United States Patent 2472292 discloses related compounds to those of the invention. Although these are described as having particular biological activity, there is no suggestion that they may have fungicidal activity.

It has now been found that a new class of compounds, pyridyliminomethylbenzene derivatives, show interesting fungicidal activity and accordingly the present invention provides the use as a fungicide of a pyridyliminomethylbenzene derivative or an acid-addition salt thereof, the derivative being a compound of general formula;-

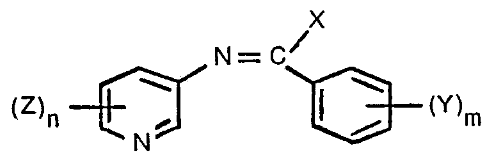

(I)

wherein x is optionally-substituted alkoxy, cycloalkoxy, alkenyloxy, alkynyloxy, aryloxy, haloaryloxy, alkoxyaryloxy, aralkyloxy, haloaralkyloxy, alkoxyaralkyloxy, optionally-substituted alkylthio, cycloalkylthio, alkenylthio, alkynylthio, arylthio, haloarylthio, alkoxyarylthio, aralkylthio, or a group of formula:

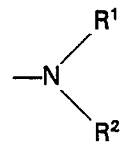

where $R^1$ and $R^2$ are independently selected from hydrogen and optionally-substituted alkyl, or $R^1$ and $R^2$ together with the interjacent nitrogen atom form a heterocyclic ring of from 5 to 7 ring atoms, one of which ring atoms may be a further heteroatom selected from nitrogen, oxygen and sulphur; m is 0, 1 or 2; Y is an alkyl, haloalkyl, alkoxy, haloalkoxy, halo, nitro, aryloxy, haloaryloxy, cyano or alkoxycarbonyl moiety; n is 0, 1 or 2; and Z is an alkyl, alkoxy or halo moiety. (It is noted that when $m = 0$ and $n = 0$ then Y and Z are respectively hydrogen atoms).

The compounds of formula I have been found to have fungicidal properties, and specific examples will be given hereinafter to illustrate the excellent activity of compounds of formula I as fungicides and the versatility thereof in terms of the wide range of fungus species which can be controlled by using such compounds.

So far as the applicants are aware the compounds of formula I defined above (and their acid addition salts), as well as compositions containing those compounds are novel. Accordingly the invention further provides a fungicidal composition comprising at least one inert carrier together with, as active ingredient, at least one fungicidally active pyridyliminomethylbenzene derivative of formula I, or an acid addition salt thereof, as defined above, and also such pyridyliminomethylbenzene derivatives *per se*. The invention further provides a method of making such a composition which comprises bringing a compound of formula I as defined above (or an acid addition salt thereof) into association with at least one inert carrier therefor.

An application of the derivatives of formula I above, or their acid addition salts, or of compositions containing such compounds may be in a method of protecting a crop from fungal attack comprising treating crops subject to or subjected to fungal attack, seeds of such crops or soil in which such crops are growing or are to be grown with such compounds or compositions.

The pyridyliminomethylbenzene derivatives of formula I are, of course, compounds capable of forming acid-addition salts, for example, salts of hydrohalic acids, particularly of hydrochloric acid, or of sulphuric acid. Such salts are also novel compounds; and they are of interest as having the fungicidal activity of the pyridyliminomethylbenzene derivatives from which they are formed (in a manner well-known per se), and, accordingly, are included within the scope of the present invention. It is also noted in regard to such compounds that desirably they should be non-phytotoxic, which can readily be determined by routine test, since normally this is a requirement of fungicidal compositions.

Referring to the compounds of formula I, the optional substituents in the optionally substituted alkyl, optionally substituted alkoxy and optionally-substituted alkylthio groups are preferably selected

from halogen, alkoxy, aryloxy, alkylthio, arylthio, alkylamino, dialkylamino, alkylidenamino and aryl moieties.

Alkyl, alkenyl, alkynyl, cycloalkyl, alkoxyaryl, and aralkyl moieties in the various substituents in formula I preferably contain up to 10 carbon atoms. Preferred aryl groups are phenyl groups. The alkyl, alkenyl and alkynyl groups may be straight-chain or branched groups.

Preferred halogen substituents are chlorine, fluorine and bromine atoms, chlorine being particularly preferred.

At present preferred compounds of Formula I from the point of view of utilising the fungicidal activity which has been found to arise from the presence of the 3′-pyridyliminomethylbenzene structure which characterises the compounds of formula I are the simpler (overall molecular structure wise) pyridyliminomethylbenzene derivatives in which n = 0. However, those additionally substituted pyridyliminomethylbenzene derivatives in which one or two of the defined substituents Z are present can be used, as desired, as fungicidally active compounds where, for example, particular characteristics, for example, particular physical properties are sought. The fungicidal activity achievable with the compounds of formula I, and particularly those in which n = 0, is especially advantageous being such as to enable those in the art readily to formulate, on the basis of the result of standard biological and physical tests, a range of fungicidal compositions having the characteristics required for particular end use applications, for example, either relatively broad or relatively specific activity fungus speciewise, activity which is transmitted through plant tissue (ie. is not confined to the surfaces tested), and, by simple variation of the nature of the defined substituents, compositions in which the active compound exhibits significant vapour pressure. Also, such compounds have the advantage of being usable in combination with other known fungicides where it is desired to provide compositions exhibiting a particular spectrum and level of activity.

When X is a group of formula —$NR^1R^2$, wherein $R^1$ and $R^2$ together with the interjacent nitrogen atom form a heterocyclic ring of from 5 to 7 ring atoms, the ring is advantageously a piperidinyl, pyrrolidinyl, piperazinyl, morpholinyl or thiamorpholinyl ring, a six-membered nitrogen-containing heterocyclic ring being preferred.

Preferred compounds of Formula I are those wherein X is alkoxy, aryloxy, alkyloxyalkoxy, alkylthio, alkylamino, substituted alkylamino, for example, amino-alkyl amino and alkoxy-alkylamino, or dialkylamino, m is 0, 1 or 2, Y is halogen and n is 0. Advantageously X is alkoxy, aryloxy, alkylthio, mono-alkylamino, di-alkylamino or a six-membered nitrogen-containing heterocyclic ring attached to the carbon atom through its nitrogen atom; and preferably X is $C_{1-6}$ alkoxy, $C_{1-6}$ alkylthio or di-($C_{1-6}$ alkyl) amino. Advantageously $(Y)_m$ is a cyano, halo or haloalkyl substituent, and in the case of a single such substituent it is preferably attached at the 4-position of the benzene ring. In such case a single halo substituent is preferred, and $(Y)_m$ is then preferably a 4-chloro-substituent as, for example, in a preferred compound: 4-chloro-(3′-pyridyl)imino-C-(isobutylthio)methylbenzene.

However, compounds in which $(Y)_m$ represents di-halo substitution are also particularly effective. In such case $(Y)_m$ is preferably dichloro-, and advantageously the chlorine atoms are in the 2,4- or 3,4-positions of the benzene ring.

The invention also provides processes for preparing the compounds of the invention. The compounds of formula I are prepared in accordance with the invention by reacting a compound of general formula:

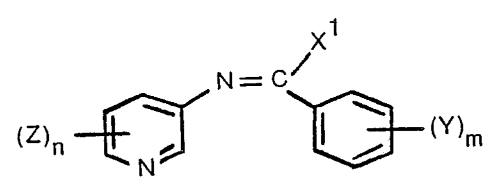

(II)

where m, n, Y and Z are all as defined above and $X^1$ is halogen, with a compound of formula

Q—X

where X as defined above and Q is hydrogen or an alkali metal atom, optionally in the presence of an acid acceptor. $X^1$ is preferably a chlorine atom.

The intermediates of formula II are themselves provided as a further aspect of the invention. They are conveniently prepared by reacting a thionyl halide with the appropriate N-(3′-pyridyl)benzamide of general formula:

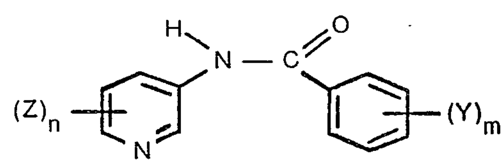

(V)

where m, n, Y and Z are as defined above.

Compounds of formula I in which X is optionally-substituted alkylthio, cycloalkylthio, alkenylthio, alkynylthio, arylthio, haloarylthio, alkoxyarylthio or aralkylthio can also be prepared by alkylation of the corresponding thioanilides.

As previously stated the salts of the novel compounds of formula I can be prepared from such compounds by methods well-known per se. Accordingly, and with the specific exemplification of the formation of the hydrochlorides hereinafter, it is considered unnecessary to describe such methods in detail since those in the art will be familiar with them.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toulene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having similar composition to that of a wettable powder but without a dispersant, and are diluted in the field with further solid carrier to give a composition usually containing $\frac{1}{2}$—10% w of active ingredient. Granules are usually prepared to have a size between 10 and 100 BS mesh (1.676—0.152 mm), and may be manufactured by agglomeration or impregnation techniques. Generally, granules will contain in addition to a carrier $\frac{1}{2}$—25% w active ingredient and 0—10% of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as deformers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the present

invention. The said emulsions may be of the water-in-oil or of the oil-in-water type, and may have a thick 'mayonnaise'-like consistency.

In order to illustrate the fungicidal activity of compounds of Formula I above the examples given hereinafter of a wide range of compounds within formula I and representative of the scope thereof include results obtained by subjecting these compounds to a variety of tests representative of the spectrum of fungus species against which fungicidal compositions are required for use. The following tests were carried out:

(a) Activity against apple powdery mildew (Podosphaera leucotricha; P.1)

The test is a direct anti-sporulant one using a foliar spray. The upper surfaces of leaves of whole apple seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer. After drying the plants are returned to a compartment at ambient temperature and humidity for up to 9 days, followed by assessment. Assessment is based on the percentage of the leaf area covered by sporulation compared with that on leaves of control plants.

(b) Activity against vine downy mildew (Plasmopera viticola; Pv.t)

The test is a translaminar protectant one using a foliar spray. The upper surfaces of leaves of whole vine plants are sprayed at a dosage of 1 kilogram of active material per hectare using a track sprayer. The lower surfaces of the leaves are then inoculated, up to 6 hours after treatment with the test compound, by spraying with an aqueous suspension containing $10^5$ zoosproangia/ml. The inoculated plants are kept for 24 hours in a high humidity compartment, 4 days at glasshouse ambient temperature and humidity and then returned for a further 24 hours to high humidity. Assessment is based on the percentage of the leaf area covered by sporulation compared with that on control leaves.

(c) Activity against vine grey mould (Botrytis cinerea; B.c)

The test is a direct eradicant one using a foliar spray. The under-surface of the detached vine leaves are inoculated by pipetting ten large drops of an aqueous suspension containing $5 \times 10^5$ conidia/ml on to them. The inoculated leaves are kept uncovered overnight during which time the fungus has penetrated the leaf and a visible necrotic lesion may be apparent where the drop was made. The infected regions are sprayed directly with a dosage of 1 kg of active material per hectare using a track sprayer. When the spray has dried the leaves are covered with petri dish lids and the disease allowed to develop under the moist conditions. The extent of the necrotic lesion beyond the original drop together with the dosage of sporulation is compared with that on control leaves.

(d) Activity against peanut leaf spot (Cercospora arachidicola; Ca)

The test is a direct eradicant one using a foliar spray. The upper surfaces of the leaves of peanut plants (12—20 cms high, in monopots) are inoculated by spraying with an aqueous suspension containing $10^5$ conidia/ml 40—43 hours prior to treatment with the test compound. The inoculated plants are kept at high humidity and then allowed to dry during the interval between inoculation and treatment by spraying at a dosage of 1 kg of active material per hectare using a track sprayer. After spraying the plants are moved to a humid compartment at 25—28°C for a further period of up to 10 days. Assessment is based on a comparison between the levels of disease on the treated and control plants.

(e) Activity against potato late blight (Phytophthora infestans; P.i.p.)

The test measures the direct protectant activity of compounds applied as a foliar spray. Tomato plants, Cultivar Ailsa Craig, 1—15 cms high, in monopots are used. The whole plant is sprayed at a dosage of 1 kilogram of active material per hectare using a track sprayer. The plant is then inoculated up to 6 hours after treatment with the test compound, by spraying with an aqueous suspension containing $5 \times 10^3$ zoozporangia/ml. The inoculated plants are kept in high humidity for 3 days. Assessment is based on comparison between the levels of disease on the treated and control plants.

(f) Activity against barley powdery mildew (Erysiphe graminis; Eg.)

The test measures the direct anti-sporulant activity of compounds applied as a foliar spray. For each compound about 40 barley seedlings were grown to the one-leaf stage in a plastic pot of sterile potting compost. Inoculation was effected by dusting the leaves with conidia of Erysiphe graminis, spp. hordei. 24 hours after inoculation the seedlings were sprayed with a solution of the compound in a mixture of acetone (50%), surfactant (0.04%) and water using a track sprayer. The rate of application was equivalent to 1 kg of active material per hectare. First assessment of disease was made 5 days after treatment when the overall level of sporulation on the treated plants were compared with that on control plants.

The extent of disease control achieved in such tests is expressed as a control rating according to criteria in which a greater than 80% disease control is given the rating 2.

Specific examples of compounds of formula I are provided by the following examples, which are provided as illustrative but in no way limitative examples. Relevant data pertaining to each of these examples is given in the appended Tables I and II. These tables include illustrative, but non-limitative, rating 2 results (where available) in the range of tests described above. The nature and extent (test spread-wise) of the results quoted demonstrates the good and useful fungicidal activity attainable using compounds of formula I as above.

Example 1

4-Chloro-(3′-pyridyl)imino-C-(isopropoxy)methylbenzene

A stirred mixture of N-(3′-pyridyl)-4-chlorobenzamide (7 g, 0.03 mole) and thionyl chloride (22.5 ml) was heated under reflux for two hours. Excess thionyl chloride was then removed *in vacuo* and the residue, 4-chloro-(3′pyridyl)imino-C(chloro)-methylbenzene hydrochloride, was suspended in dry dimethoxyethane (75 ml). A solution formed by dissolving sodium (2.43 g, 0.105 mole) in dry isopropanol (100 ml) was poured into the suspension and the resulting mixture was stirred for one hour at room temperature and then heated under reflux for sixteen hours. The solvent component of the mixture was removed *in vacuo,* and the residue was treated with diethyl ether. The ethereal solution was washed twice with water and then dried over anhydrous magnesium sulphate. After removal of the ether, the residue was subjected to chromatography on a silica gel column, eluting with diethyl ether/hexane (1:1). The title product was obtained as a colourless solid (yield, 53%) m.p. 65—67°C. (Ref: WL No. 81910).

A greater than 80% disease control was achieved in the Pv.t test.

Example 2

4-Chloro-(3′-pyridyl)imino-C-(isopropylthio)methylbenzene

A stirred mixture of N-(3′-pyridyl)-4-chlorobenzamide (7 g, 0.03 mole) and thionyl chloride (22.5 ml) was heated under reflux for two hours. Excess thionyl chloride was removed *in vacuo*, and the residue, 4-chloro-(3′-pyridyl)imino-C-(chloro)methylbenzene hydrochloride, was treated with dry pyridine (100 ml). Isopropyl mercaptan (4.56 g, 0.06 mole) was added and the mixture was stirred and heated in an oil-bath at a temperature in the range from 100—110°C for sixteen hours. The solvent and other volatile components of the mixture were removed *in vacuo,* and the residue was treated with diethyl ether. The ethereal solution was washed three times with water and then dried over anhydrous magnesium sulphate. After removal of the ether, the residue was subjected to chromatography on a silica gel column, eluting with diethyl ether/hexane (1:1). The title product was obtained as a yellow solid (yield, 70%) m.p. 55.5—57°C. (Ref: WL No. 81963)

A greater than 80% disease control was achieved in the E.g. test.

Example 3

4-Chloro-(3′-pyridyl)imino-C-(di-n-butylamino)methylbenzene

A stirred mixture of N-(3′-pyridyl)-4-chlorobenzamide (7 g, 0.03 mole) and thionyl chloride (22.5 ml) was heated under reflux for two hours. Excess thionyl chloride was removed *in vacuo* and the residue, 4-chloro-(3′-pyridyl)imino-C-(chloro)methylbenzene hydrochloride, was suspended in dry dimethoxyethane (125 ml). Dry di-*n*-butylamino (19.4 g, 0.15 mole) was added and the resulting mixture was stirred at ambient temperature overnight. The solvent component of the mixture was removed *in vacuo,* and the residue was treated with diethyl ether. The ethereal solution was washed twice with water and then dried over anhydrous magnesium sulphate. After removal of the ether, the residue was subjected to chromatography on a silica gel column, eluting with diethyl ether, and again on a silica gel column, eluting with diethyl ether/methylene dichloride (1:1). The title product was obtained as a viscous oil (yield 28%). (Ref: WL No. 83216)

A greater than 80% disease control was achieved in the E.g. test.

| | | | | |
|---|---|---|---|---|
| Analysis: calculated for $N_3ClC_{20}H_{26}$ | : | C 69.9; | H 7.6; | N 12.2% |
| found | : | C 69.9; | H 8.1; | N 12.0% |

Examples 4 to 39

The compounds listed in Tables I and II were prepared by similar methods to those described in Examples 1 to 3:

TABLE I

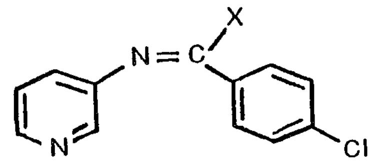

| WL No. | Example | X | Yield % | mp °C | Analysis % | C | H | N | Greater than 80% disease control achieved in the below-indicated test |
|---|---|---|---|---|---|---|---|---|---|
| 83420 | 4 | $-O(CH_2)_3CH_3$ | 62 | | $N_2OClC_{16}H_{17}$ | 66.6 | 5.9 | 9.7 | E.g |
| | | | | | Found | 66.6 | 6.3 | 9.5 | |
| 83463 | 5 | $-OCH_2CH(CH_3)_2$ | 22 | 34–5 | $N_2OClC_{16}H_{17}$ | 66.6 | 5.9 | 9.7 | E.g. |
| | | | | | Found | 66.4 | 6.0 | 9.4 | |
| 83464 | 6 | $-OCH(CH_3)CH_2CH_3$ | 86 | | $N_2OClC_{16}H_{17}$ | 66.6 | 5.9 | 9.7 | B.c |
| | | | | | Found | 66.5 | 6.0 | 9.5 | |
| 83469 | 7 | $-OCH_3$ | 54 | 51 | $N_2OClC_{13}H_{11}$ | 63.3 | 4.5 | 11.4 | E.g. |
| | | | | | Found | 62.2 | 4.4 | 11.3 | |
| 83470 | 8 | $-OCH_2CH_2OCH_3$ | 80 | 57 | $N_2O_2ClC_{15}H_{15}$ | 61.9 | 5.2 | 9.6 | |
| | | | | | Found | 60.9 | 5.1 | 9.7 | |

TABLE I (Continued)

| WL No. | Example | X | Yield % | mp °C | | Analysis % C | H | N | Greater than 80% disease control achieved in the below-indicated test |
|---|---|---|---|---|---|---|---|---|---|
| 83555 | 9 | $-S(CH_2)_3CH_3$ | 48 | | $N_2SClC_{16}H_{17}$ | 63.1 | 5.6 | 9.2 | E.g. |
| | | | | | Found | 63.4 | 5.7 | 8.0 | |
| 83641 | 10 | $-SCH(CH_3)CH_2CH_3$ | 55 | | $N_2SClC_{16}H_{17}$ | 63.1 | 5.6 | 9.2 | Pv.t |
| | | | | | Found | 63.0 | 5.7 | 9.1 | |
| 83642 | 11 | $-SCH_2CH_2CH_3$ | 47 | | $N_2SClC_{15}H_{15}$ | 62.0 | 5.2 | 9.6 | E.g |
| | | | | | Found | 62.6 | 5.4 | 9.6 | |
| 83676 | 12 | $-SC(CH_3)_3$ | 37 | 97 | $N_2SClC_{16}H_{17}$ | 63.1 | 5.6 | 9.2 | E.g. |
| | | | | | Found | 63.2 | 5.7 | 9.2 | |
| 83677 | 13 | $-SCH_2CH(CH_3)_2$ | 76 | | $N_2SClC_{16}H_{17}$ | 63.1 | 5.6 | 9.2 | E.g. |
| | | | | | Found | 63.9 | 6.2 | 9.3 | |

TABLE II

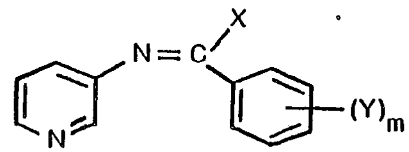

| WL No. | Example | X | Y | mp. (°C) | Analysis % | C | H | N | Greater than 80% disease control achieved in the below-indicated test |
|---|---|---|---|---|---|---|---|---|---|
| 83868 | 14 | 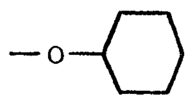 | 4–Cl | oil | Found | 68.3 | 6.2 | 8.7 | P.i.p |
| | | | | | $N_2OClC_{18}H_{19}$ | 68.68 | 6.04 | 8.9 | |
| 83939 | 15 | $-OCH_2CH_3$ | 4–Cl | 53 | Found | 65.2 | 5.1 | 10.9 | E.g. |
| | | | | | $N_2OClC_{14}H_{13}$ | 64.49 | 4.99 | 10.25 | |
| 83940 | 16 | 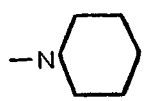 | 4–Cl | 74.5 | Found | 67.4 | 6.1 | 14.1 | E.g. |
| | | | | | $N_3ClC_{17}H_{18}$ | 68.11 | 6.01 | 14.02 | |
| 84331 | 17 | $-NHCH_2CH_2OCH_3$ | 4–Cl | 78 | Found | 62.14 | 5.6 | 14.2 | |
| | | | | | $N_3OClC_{15}H_{16}$ | 62.18 | 5.53 | 14.51 | |

TABLE II (Continued)

| WL No. | Example | X | Y | mp. (°C) | | Analysis % C | H | N | Greater than 80% disease control achieved in the below-indicated test |
|---|---|---|---|---|---|---|---|---|---|
| 84680 | 18 | 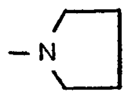 | 4–Cl | 102 | Found | 67.0 | 5.7 | 14.5 | |
| | | | | | $N_3ClC_{16}H_{16}$ | 67.3 | 5.6 | 14.7 | |
| 85002 | 19 | $-NHCH_2CH_2N(C_2H_5)_2$ | 4–Cl | oil | Found | 65.4 | 7.3 | 16.4 | |
| | | | | | $N_4ClC_{18}H_{23}$ | 65.36 | 6.96 | 16.94 | |
| 85037 | 20 | $-SC(CH_3)_3$ | 2,4–$Cl_2$ | 90 | Found | 56.8 | 4.8 | 8.2 | B.c. |
| | | | | | $N_2SCl_2C_{16}H_{16}$ | 56.64 | 4.72 | 8.26 | |
| 85500 | 21 | $-SC(CH_3)_3$ | 3,4–$Cl_2$ | 85–86 | Found | 56.1 | 4.8 | 8.1 | P.I |
| | | | | | $N_2SCl_2C_{16}H_{16}$ | 56.64 | 4.72 | 8.26 | |
| 85501 | 22 | 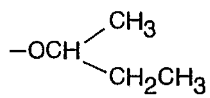 | 2,4–$Cl_2$ | oil | Found | 60.1 | 5.1 | 8.6 | C.a |
| | | | | | $N_2OCl_2C_{16}H_{16}$ | 59.44 | 4.95 | 8.67 | |
| 85575 | 23 | 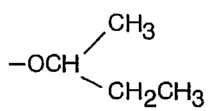 | 3,4–$Cl_2$ | oil | Found | 60.1 | 5.4 | 8.6 | P.I |
| | | | | | $N_2OCl_2C_{16}H_{16}$ | 59.44 | 4.95 | 8.67 | |
| 85576 | 24 | $-SCH_2CH(CH_3)_2$ | 2,4–$Cl_2$ | oil | Found | 57.3 | 5.0 | 8.3 | E.g. |
| | | | | | $N_2SCl_2C_{16}H_{16}$ | 56.64 | 4.72 | 8.26 | |
| 85777 | 25 | $-SCH_2CH_2CH_3$ | 2,4–$Cl_2$ | oil | Found | 56.3 | 4.7 | 8.5 | P.i.p |
| | | | | | $N_2SCl_2C_{15}H_{14}$ | 55.4 | 4.3 | 8.62 | |

TABLE II (Continued)

| WL No. | Example | X | Y | mp. (°C) | Analysis % | C | H | N | Greater than 80% disease control achieved in the below-indicated test |
|---|---|---|---|---|---|---|---|---|---|
| 85778 | 26 | 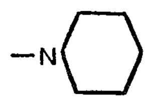 | 3,4–$Cl_2$ | 76 | Found<br>$N_3Cl_2C_{17}H_{17}$ | 61.2<br>61.07 | 5.1<br>5.09 | 12.6<br>12.57 | P.l |
| 85779 | 27 | –$SC(CH_3)3$ | 4–F | 53 | Found<br>$N_2SFC_{16}H_{17}$ | 66.9<br>66.67 | 6.0<br>5.9 | 10.2<br>9.7 | P.l |
| 85780 | 28 | 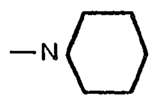 | 2,4–$Cl_2$ | 68 | Found<br>$N_3Cl_2C_{17}H_{17}$ | 61.4<br>61.07 | 5.3<br>5.09 | 12.6<br>12.57 | P.i.p |
| 86160 | 29 | 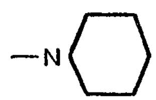 | 4–F | oil | Found<br>$N_3FC_{17}H_{18}$ | 71.8<br>72.08 | 7.0<br>6.36 | 14.8<br>14.84 | B.c |
| 86161 | 30 | –$SCH(CH_3)_2$ | 2,4–$Cl_2$ | 43–4 | Found<br>$N_2SCl_2C_{15}H_{14}$ | 55.4<br>55.4 | 4.3<br>4.3 | 8.6<br>8.6 | E.g |
| 86350 | 31 | –$O(CH_2)_3CH_3$ | 2,4–$Cl_2$ | oil | Found<br>$N_2OCl_2C_{16}H_{16}$ | 60.4<br>59.44 | 5.5<br>4.95 | 8.5<br>8.67 | E.g |
| 86351 | 32 | –$OCH(CH_3)_2$ | 2,4–$Cl_2$ | 43 | Found<br>$N_2OCl_2C_{15}H_{14}$ | 58.9<br>58.3 | 4.9<br>4.5 | 9.1<br>9.1 | B.c. |

TABLE II (Continued)

| WL No. | Example | X | Y | mp. (°C) | | Analysis % C | H | N | Greater than 80% disease control achieved in the below-indicated test |
|---|---|---|---|---|---|---|---|---|---|
| 86403 | 33 | 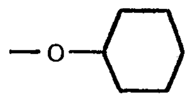 | 2,4–$Cl_2$ | oil | Found<br>$N_2OCl_2C_{18}H_{18}$ | 62.1<br>61.9 | 5.5<br>5.16 | 7.8<br>8.02 | E.g |
| 86404 | 34 | 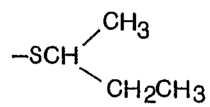 | 2,4–$Cl_2$ | oil | Found<br>$N_2SCl_2C_{16}H_{16}$ | 56.7<br>56.64 | 5.0<br>4.72 | 8.2<br>8.26 | B.c |
| 86561 | 35 | $-SC(CH_3)_3$ | H | 80–81 | Found<br>$N_2SC_{16}H_{18}$ | 71.0<br>71.1 | 6.6<br>6.7 | 10.4<br>10.4 | |
| 86722 | 36 | $-SC(CH_3)_3$ | 4-cyano | 96.5–<br>97.5 | Found<br>$N_3SC_{17}H_{17}$ | 68.5<br>69.15 | 5.9<br>5.76 | 14.2<br>14.24 | |
| 86723 | 32 | $-OCH_2CH(CH_3)_3$ | 4–F | oil | Found<br>$N_2OFC_{16}H_{17}$ | 71.7<br>70.59 | 7.3<br>6.25 | 9.4<br>10.29 | |
| 86724 | 38 | $-OCH_2CH(CH_3)_2$ | H | oil | Found<br>$N_2OC_{16}H_{18}$ | 75.7<br>75.59 | 7.4<br>7.08 | 10.9<br>11.02 | |
| 86741 | 39 | 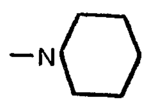 | H | oil | Found<br>$N_3C_{17}H_{19}$ | 75.6<br>76.98 | 7.1<br>7.17 | 15.4<br>15.85 | |

**Claims for the Contracting States: BE CH DE FR GB IT NL SE**

1. Use as a fungicide of a pyridyliminomethylbenzene derivative or an acid-addition salt thereof, said derivative being a compound of general formula:-

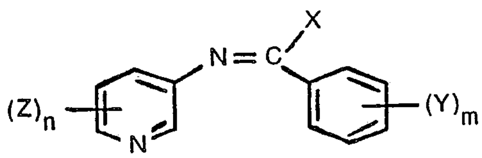

(I)

wherein X is optionally substituted alkoxy, cycloalkoxy, alkenyloxy, alkynyloxy, aryloxy, haloaryloxy, alkoxyaryloxy, aralkyloxy, haloaralkyloxy, alkoxyaralkyloxy, optionally substituted alkylthio, cycloalkylthio, alkenylthio, alkynylthio, arylthio, haloarylthio, alkoxyarylthio, aralkylthio, or a group of formula:

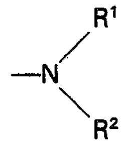

where $R^1$ and $R^2$ are independently selected from hydrogen and optionally substituted alkyl, or $R^1$ and $R^2$ together with the interjacent nitrogen atom form a heterocyclic ring of from 5 to 7 ring atoms, one of which ring atoms may be a further heteroatom selected from nitrogen, oxygen and sulphur,

m is 0, 1 or 2;

Y is an alkyl, haloalkyl, alkoxy, haloalkoxy, halo, nitro, aryloxy, haloaryloxy, cyano or alkoxycarbonyl moiety;

n is 0, 1 or 2; and

Z is an alkyl, alkoxy or halo moiety.

2. Use as claimed in Claim 1 wherein the derivative is a compound of formula I, wherein X is alkoxy, alkyloxyalkoxy, alkylthio, alkylamino or dialkylamino; m is 0, 1 or 2; Y is halogen; and n is 0.

3. Use as claimed in Claim 1 or 2, wherein the derivative is a compound of formula I, wherein X is $C_{1-6}$ alkoxy, $C_{1-6}$ alkylthio or di-($C_{1-6}$ alkyl)amino, Y is halogen and n is 0.

4. Use as claimed in any one of Claims 1 to 3 wherein the derivative is a compound of formula I, wherein $(Y)_m$ is a 4-halo substituent.

5. Use as claimed in Claim 4, wherein $(Y)_m$ is a 4-chloro substituent.

6. Use as claimed in Claim 1, wherein the derivative is a compound of formula I, wherein X is cycloalkyloxy, amino-alkylamino or alkoxylalkylamino; m is 1 or 2; Y is halogen; and n is 0.

7. Use as claimed in Claim 6, wherein Y is chlorine.

8. Use as claimed in any one of Claims 1 to 3, 6 and 7, wherein the derivative is a compound of formula I, wherein Y is chlorine and m is 2, the substituents $(Y)_m$ being in the 2,4- or 3,4-positions of the benzene ring.

9. Use as claimed in Claim 1, wherein the derivative is a compound of formula I, wherein X is alkoxy, aryloxy, alkylthio, mono-alkylamino, dialkylamino or a six-membered nitrogen-containing heterocyclic ring attached to the carbon atom through its nitrogen atom; m is 0, 1 or 2; and Y is halogen, haloalkyl or cyano; and n is 0.

10. A fungicidal composition comprising at least one inert carrier together with, as active ingredient, at least one fungicidally active pyridyliminomethylbenzene derivative of formula I, or an acid addition salt thereof, as defined in any of the preceding claims.

11. A pyridyliminomethylbenzene derivative of formula I as defined in claim 1, or an acid addition salt thereof.

12. A process for preparing a compound of formula I as defined in Claim 1 which comprises reacting a compound of general formula

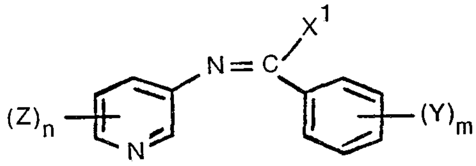

(II)

where m, n, Y and Z are all as defined in Claim 1 and $X^1$ is halogen, with a compound of formula

Q—X

wherein X is as defined in Claim 1 and Q is hydrogen or an alkali metal atom, optionally in the presence of an acid acceptor.

13. A compound of general formula:

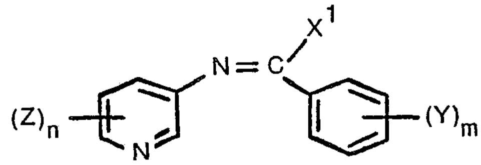

wherein $X^1$ is halogen; m is 0, 1 or 2; Y is an alkyl, haloalkyl, alkoxy, haloalkoxy, halo, nitro, aryloxy; haloaryloxy, cyano or alkoxycarbonyl moiety; n is 0, 1 or 2; and Z is an alkyl, alkoxy or halo moiety.

**Claims for the Contracting State: AT**

1. Use as a fungicide of a pyridyliminomethylbenzene derivative or an acid-addition salt thereof, said derivative being a compound of general formula:-

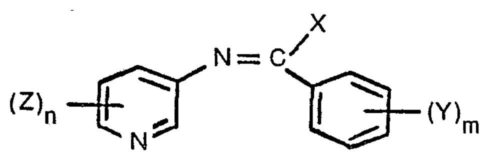

(I)

wherein X is optionally substituted alkoxy, cycloalkoxy, alkenyloxy, alkynyloxy, aryloxy, haloaryloxy, alkoxyaryloxy, aralkyloxy, haloaralkyloxy, alkoxyaralkyloxy, optionally substituted alkylthio, cycloalkylthio, alkenylthio, alkynylthio, arylthio, haloarylthio, alkoxyarylthio, aralkylthio, or a group of formula:

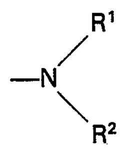

where $R^1$ and $R^2$ are independently selected from hydrogen and optionally substituted alkyl, or $R^1$ and $R^2$ together with the interjacent nitrogen atom form a heterocyclic ring of from 5 to 7 ring atoms, one of which ring atoms may be a further heteroatom selected from nitrogen, oxygen and sulphur,

m is 0, 1 or 2;

Y is an alkyl, haloalkyl, alkoxy, haloalkoxy, halo, nitro, aryloxy, haloaryloxy, cyano or alkoxycarbonyl moiety;

n is 0, 1 or 2; and

Z is an alkyl, alkoxy or halo moiety.

2. Use as claimed in Claim 1 wherein the derivative is a compound of formula I, wherein X is alkoxy, alkoxyalkoxy, alkylthio, alkylamino or dialkylamino; m is 0, 1 or 2; Y is halogen; and n is 0.

3. Use as claimed in Claim 1 or 2, wherein the derivative is a compound of formula I, wherein X is $C_{1-6}$ alkoxy, $C_{1-6}$ alkylthio or di-($C_{1-6}$ alkyl)amino, Y is halogen and n is 0.

4. Use as claimed in any one of Claims 1 to 3 wherein the derivative is a compound of formula I, wherein $(Y)_m$ is a 4-halo substituent.

5. Use as claimed in Claim 4, wherein $(Y)_m$ is a 4-chloro substituent.

6. Use as claimed in Claim 1, wherein the derivative is a compound of formula I, wherein X is cycloalkyloxy, aminoalkylamino or alkoxylalkylamino; m is 1 or 2; or Y is halogen; and n is 0.

7. Use as claimed in Claim 6, wherein Y is chlorine.

8. Use as claimed in any one of Claims 1 to 3, 6 and 7, wherein the derivative is a compound of formula I, wherein Y is chlorine and m is 2, the substituents $(Y)_m$ being in the 2,4- or 3,4-positions of the benzene ring.

9. Use as claimed in Claim 1, wherein the derivative is a compound of formula I, wherein X is alkoxy, aryloxy, alkylthio, mono-alkylamino, dialkylamino or a six-membered nitrogen-containing heterocyclic ring attached to the carbon atom through its nitrogen atom; m is 0, 1 or 2; and Y is halogen, haloalkyl or cyano; and n is 0.

10. A fungicidal composition comprising at least one inert carrier together with, as active ingredient, at least one fungicidally active pyridyliminomethylbenzene derivative of formula I, or an acid addition salt thereof, as defined in any of the preceding claims.

11. A process for preparing a compound of formula I as defined in Claim 1, which comprises reacting a compound of general formula

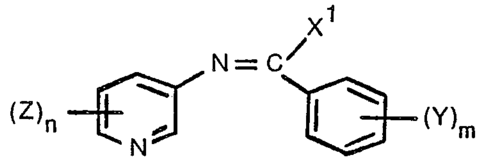

(II)

wherein m, n, Y and Z are all as defined in Claim 1 and $X^1$ is halogen, with a compound of formula

Q—X

wherein X is as defined in Claim 1 and Q is hydrogen or an alkali metal atom, optionally in the presence of an acid acceptor.

**Patentansprüche für die Vertragsstaaten: BE CH DE FR GB IT NL SE**

1. Verwendung eines Pyridyliminomethylbenzol-Derivats oder eines Säure-Additionssalzes hievon als Fungizid, welches Derivat eine Verbindung der allgemeinen Formel:

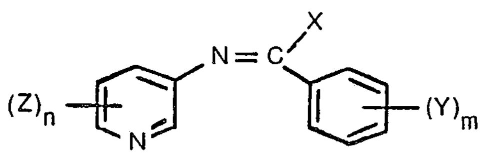

(I)

ist, worin X für gegebenenfalls substituiertes Alkoxy, Cycloalkoxy, Alkenyloxy, Alkinyloxy, Aryloxy, Halogenaryloxy, Alkoxyaryloxy, Aralkyloxy, Halogenaralkyloxy, Alkoxyaralkyloxy, gegebenenfalls substituiertes Alkylthio, Cycloalkylthio, Alkenylthio, Alkinylthio, Arylthio, Halogenarylthio, Alkoxyarylthio, Aralkylthio oder für eine Gruppe der Formel:

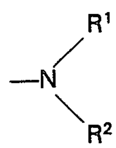

steht, worin $R^1$ und $R^2$ unabhängig voneinander unter Wasserstoff und gegebenenfalls substituiertem Alkyl ausgewählt sind oder $R^1$ und $R^2$ gemeinsam mit dem dazwischenliegenden Stickstoffatom einen heterocyclischen Ring mit von 5 bis 7 Ringatomen bilden, wobei eines dieser Ringatome ein weiteres Heteroatom, ausgewählt unter Stickstoff, Sauerstoff und Schwefel, sein kann;

m den Wert Null, 1 oder 2 hat;

Y für einen Alkyl-, Halogenalkyl-, Alkoxy-, Halogenalkoxy-, Halogen-, Nitro-, Aryloxy-, Halogenaryloxy-, Cyano oder Alkoxycarbonylrest steht;

n den Wert Null, 1 oder 2 hat; und

Z für einen Alkyl-, Alkoxy- oder Halogenrest steht.

2. Verwendung nach Anspruch 1, worin das Derivat eine Verbindung der Formel (I) ist, in welcher X Alkoxy, Alkyloxyalkoxy, Alkylthio, Alkylamino oder Dialkylamino ist; m den Wert Null, 1 oder 2 hat; Y Halogen bedeutet; und n den Wert Null hat.

3. Verwendung nach Anspruch 1 oder 2, worin das Derivat eine Verbindung der Formel (I) ist, in welcher X $C_{1-6}$-Alkoxy, $C_{1-6}$-Alkylthio oder Di($C_{1-6}$-alkyl)amino bedeutet; Y Halogen ist und n den Wert Null hat.

4. Verwendung nach einem der Ansprüche 1 bis 3, worin das Derivat eine Verbindung der Formel (I) ist, in welcher $(Y)_m$ ein 4-Halogen-Substituent ist.

5. Verwendung nach Anspruch 4, worin $(Y)_m$ ein 4-Chlor-Substituent ist.

6. Verwendung nach Anspruch 1, worin das Derivat eine Verbindung der Formel (I) ist, in welcher X für Cycloalkyloxy, Aminoalkylamino oder Alkoxyalkylamino steht; m den Wert 1 oder 2 hat; Y Halogen bedeutet; und n den Wert Null hat.

7. Verwendung nach Anspruch 6, worin Y Chlor bedeutet.

8. Verwendung nach einem der Ansprüche 1 bis 3, 6 und 7, worin das Derivat eine Verbindung der Formel (I) ist, in welcher Y Chlor bedeutet und m den Wert 2 hat, wobei die Substituenten $(Y)_m$ in den 2,4- oder 3,4-Stellungen des Benzolringes vorliegen.

9. Verwendung nach Anspruch 1, worin das Derivat eine Verbindung der Formel (I) ist, worin X für Alkoxy, Aryloxy, Alkylthio, Monoalkylamino, Dialkylamino oder einen sechsgliedrigen, Stickstoff enthaltenden heterocyclischen Ring steht, der an das Kohlenstoffatom über sein Stickstoffatom gebunden ist; m den Wert Null, 1 oder 2 hat; Y für Halogen, Halogenalkyl oder Cyano steht; und n den Wert Null hat.

10. Eine fungizide Zusammensetzung, umfassend wenigstens einen inerten Träger gemeinsam mit, als aktivem Bestandteil, wenigstens einem fungizid wirksamen Pyridyliminomethylbenzol-Derivat der Formel (I) oder eines Säure-Additionssalzes hievon, wie in einem der vorstehenden Ansprüche definiert.

11. Ein Pyridyliminobenzol-Derivat der Formel (I) wie in Anspruch 1 definiert, oder ein Säure-Additionssalz hievon.

12. Ein Verfahren zur Herstellung einer Verbindung der Formel (I), wie in Anspruch 1 definiert, umfassend ein Umsetzen einer Verbindung der allgemeinen Formel

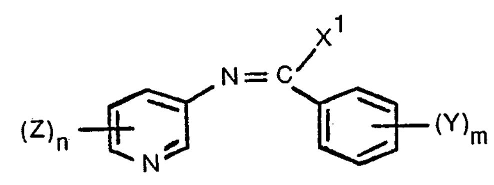

(II)

worin m, n, Y und Z alle wie in Anspruch 1 definiert sind und $X^1$ für Halogen steht, mit einer Verbindung der Formel

Q—X,

worin X wie in Anspruch 1 definiert ist und Q für Wasserstoff oder ein Alkalimetallatom steht, gewünschtenfalls in Gegenwart eines Säureakzeptors.

13. Eine Verbindung der allgemeinen Formel

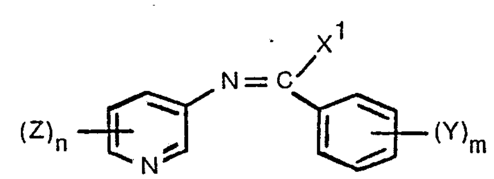

worin $X^1$ für Halogen steht; m den Wert Null, 1 oder 2 hat; Y einen Alkyl-, Halogenalkyl-, Alkoxy-, Halogenalkoxy, Halogen-, Nitro-, Aryloxy, Halogenaryloxy-, Cyan- oder Alkoxycarbonylrest bedeutet; n den Wert Null, 1 oder 2 hat; und Z für einen Alkyl-, Alkoxy- oder Halogenrest steht.

**Patentansprüche für den Vertragsstaat: AT**

1. Verwendung eines Pyridyliminomethylbenzol-Derivats oder eines Säure-Additionssalzes hievon als Fungizid, welches Derivat eine Verbindung der allgemeinen Formel:

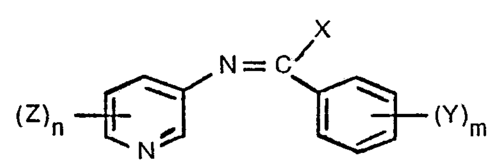

(I)

ist, worin X für gegebenenfalls substituiertes Alkoxy, Cycloalkoxy, Alkenyloxy, Alkinyloxy, Aryloxy, Halogenaryloxy, Alkoxyaryloxy, Aralkyloxy, Halogenaralkyloxy, Alkoxyaralkyloxy, gegebenenfalls substituiertes Alkylthio, Cycloalkylthio, Alkenylthio, Alkinylthio, Arylthio, Halogenarylthio, Alkoxyarylthio, Aralkylthio oder für eine Gruppe der Formel:

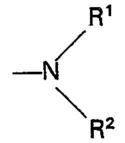

steht, worin $R^1$ und $R^2$ unabhängig voneinander unter Wasserstoff und gegebenenfalls substituiertem Alkyl ausgewählt sind, oder $R^1$ und $R^2$ gemeinsam mit dem dazwischenliegenden Stickstoffatom einen heterocyclischen Ring mit von 5 bis 7 Ringatomen bilden, wobei eines dieser Ringatome ein weiteres Heteroatom, ausgewählt unter Stickstoff, Sauerstoff und Schwefel, sein kann;

m den Wert Null, 1 oder 2 hat;

Y für einen Alkyl-, Halogenalkyl-, Alkoxy-, Halogenalkoxy-, Halogen-, Nitro-, Aryloxy-, Halogenaryloxy-, Cyano- oder Alkoxycarbonylrest steht;

n den Wert Null, 1 oder 2 hat; und

Z für einen Alkyl-, Alkoxy- oder Halogenrest steht.

2. Verwendung nach Anspruch 1, worin das Derivat eine Verbindung der Formel (I) ist, inwelcher X Alkoxy, Alkyloxyalkoxy, Alkylthio, Alkylamino oder Dialkylamino ist; m den Wert Null, 1 oder 2 hat; Y Halogen bedeutet; und n den Wert Null hat.

3. Verwendung nach Anspruch 1 oder 2, worin das Derivat eine Verbindung der Formel (I) ist, in welcher X $C_{1-6}$/Alkoxy, $C_{1-6}$-Alkylthio oder Di($C_{1-6}$-alkyl)amino bedeutet; Y Halogen ist und n den Wert Null hat.

4. Verwendung nach einem der Ansprüche 1 bis 3, worin das Derivat eine Verbindung der Formel (I) ist, in welcher $(Y)_m$ ein 4-Halogen-Substituent ist.

5. Verwendung nach Anspruch 4, worin $(Y)_m$ ein 4-Chlor-Substituent ist.

6. Verwendung nach Anspruch 1, worin das Derivat eine Verbindung der Formel (I) ist, in welcher X für Cycloalkyloxy, Aminoalkylamino oder Alkoxyalkylamino steht; m den Wert 1 oder 2 hat; Y Halogen bedeutet; und n den Wert Null hat.

7. Verwendung nach Anspruch 6, worin Y Chlor bedeutet.

8. Verwendung nach einem der Ansprüche 1 bis 3, 6 und 7, worin das Derivat eine Verbindung der Formel (I) ist, in welcher Y Chlor bedeutet und m den Wert 2 hat, wobei die Substituenten$(Y)_m$ in den 2,4- oder 3,4-Stellungen des Benzolringes vorliegen.

9. Verwendung nach Anspruch 1, worin das Derivat eine Verbindung der Formel (I) ist, worin X für Alkoxy, Aryloxy, Alkylthio, Monoalkylamino, Dialkylamino oder einen sechsgliedrigen, Stickstoff enthaltenden heterocyclischen Ring steht, der an das Kohlenstoffatom über sein Stickstoffatom gebunden ist; m den Wert Null, 1 oder 2 hat; Y für Halogen, Halogenalkyl oder Cyano steht; und n den Wert Null hat.

10. Eine fungizide Zusammensetzung, umfassend wenigstens einen inerten Träger gemeinsam mit, als aktivem Bestandteil, wenigstens einem fungizid wirksamen Pyridyliminomethylbenzol-Derivat der Formel (I) oder eines Säure-Additionssalzes hievon, wie in einem der vorstehenden Ansprüche definiert.

11. Ein Verfahren zur Herstellung einer Verbindung der Formel (I), wie in Anspruch 1 definiert, umfassend ein Umsetzen einer Verbindung der allgemeinen Formel

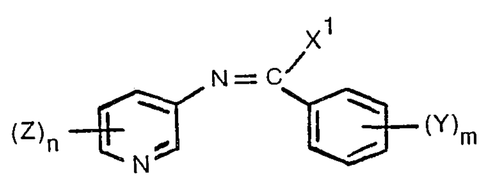

(II)

worin m, n, Y und Z alle wie in Anspruch 1 definiert sind und $X^1$ für Halogen steht, mit einer Verbindung der Formel

Q—X,

worin X wie in Anspruch 1 definiert ist und Q für Wasserstoff oder ein Alkalimetallatom steht, gewünschtenfalls in Gegenwart eines Säureakzeptors.

**Revendications pour les Etats contractants: BE CH DE FR GB IT NL SE**

1. Emploi comme fongicide d'un dérivé de pyridyliminométhylbenzène ou d'un de ses sels d'addition d'acides, ledit dérivé étant un compose de formule générale:

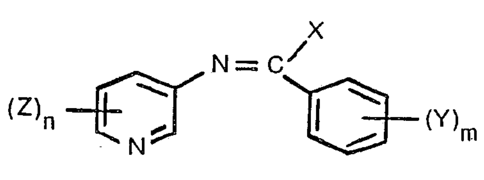

(I)

dans laquelle X est un alcoxy éventuellement substitué, un cycloalcoxy, un alcényloxy, un alcynyloxy, un aryloxy, un halogénoaryloxy, un alcoxyaryloxy, un aralkyloxy, un halogénoaralkyloxy, un alcoxy-aralkyloxy, un alkylthio éventuellement substitué, un cycloalkylthio, un alcénylthio, un alcynylthio, un arylthio, un halogénoarylthio, un alcoxyarylthio, un aralkylthio ou un groupe de formule:

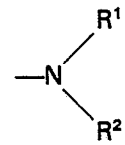

dans laquelle $R^1$ et $R^2$ sont choisis indépendamment parmi un hydrogène et un alkyle éventuellement substitué, ou $R^1$ et $R^2$ forment ensemble, avec l'atome d'azote intermédiaire, un noyau hétérocyclique de 5 à 7 atomes cycliques, un de ces atomes cycliques pouvant être un autre hétéroatome choisi parmi l'azote, l'oxygène et le soufre,

m est 0, 1 ou 2;

Y est un fragment alkyle, halogénoalkyle, alcoxy, halogénoalcoxy, halogéno, nitro, aryloxy, halogénoaryloxy, cyano ou alcoxycarbonyle;

n est 0, 1 ou 2; et

Z est un fragment alkyle, alcoxy ou halogéno.

2. Emploi comme revendiqué dans la revendication 1, dans lequel le dérivé est un composé de formule I où X est un alcoxy, un alcoxyalcoxy, un alkylthio, un alkylamino ou un dialkylamino; m est 0, 1 ou 2; Y est un halogène; et n est 0.

3. Emploi comme revendiqué dans la revendication 1 ou 2, dans lequel le dérivé est un composé de formule I où X est un alcoxy en $C_{1-6}$, un alkylthio en $C_{1-6}$ ou un di-(alkyl en $C_{1-6}$)amino, Y est un halogène et n est 0.

4. Emploi comme revendiqué dans l'une quelconque des revendications 1 à 3, où le dérivé est un compose de formule I, où $(Y)_m$ est un substituant 4-halogéno.

5. Emploi comme revendiqué dans la revendication 4, où $(Y)_m$ est un substituant 4-chloro.

6. Emploi comme revendiqué dans la revendication 1, où le dérivé est un composé de formule I, où X est un cycloalcoxy, un aminoalkylamino ou un alcoxyalkylamino; m est 1 ou 2; Y est un halogène et n est 0.

7. Emploi comme revendiqué dans la revendication 6 où X est un chlore.

8. Emploi comme revendiqué dans l'une quelconque des revendications 1 à 3, 6 et 7, où le dérivé est un composé de formule I où Y est un chlore et m est 2, les substituants $(Y)_m$ étant dans les positions 2,4 ou 3,4 du noyau benzène.

9. Emploi comme revendiqué dans la revendication 1, où le dérivé est un composé de formule I où X est un alcoxy, un aryloxy, un alkylthio, un monoalkylamino, un dialkylamino ou un noyau hétérocyclique azoté hexagonal fixé à l'atome de carbone par son atome d'azote; m est 0, 1 ou 2; Y est un halogène, un halogénoalkyle ou un cyano et n est 0.

10. Une composition fongicide comprenant au moins un support inerte avec, comme ingrédient actif, au moins un dérivé de pyridyliminométhylbenzène à activité fongicide de formule I, ou un de ses sels d'addition d'acides, comme défini dans l'une quelconque des revendications précédentes.

11. Un dérivé de pyridyliminométhylbenzène de formule I, comme défini dans la revendication 1 ou un de ses sels d'addition d'acides.

12. Un procédé pour préparer un composé de formule I, comme défini dans la revendication 1, qui comprend la réaction d'un composé de formule générale

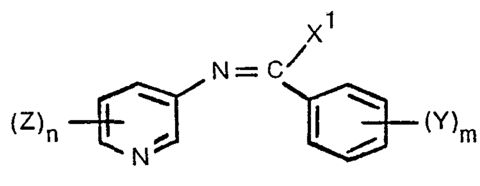

(II)

dans laquelle m, n, Y et Z sont tous comme défini dans la revendication 1, et $X^1$ est un halogène, avec un composé de formule

$$Q{-}X$$

où X est comme défini dans la revendication 1 et Q est un atome d'hydrogène ou un atome de métal alcalin, éventuellement en présence d'un accepteur d'acide.

13. Un composé de formule générale:

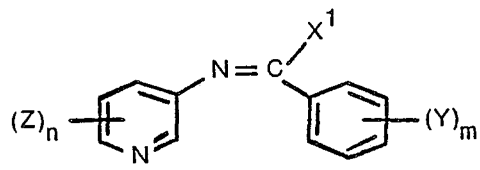

dans laquelle $X^1$ est un halogène; m est 0, 1 ou 2; Y est un fragment alkyle, halogénoalkyle, alcoxy, halogénoalcoxy, halogéno, nitro, aryloxy, halogénoaryloxy, cyano ou alcoxycarbonyle; n est 0, 1 ou 2; et Z est un fragment alkyle, alcoxy ou halogéno.

**Revendications pour l'Etat contractant: AT**

1. Emploi comme fongicide d'un dérivé de pyridyliminométhylbenzène ou d'un de ses sels d'addition d'acides, ledit dérivé étant un composé de formule générale:

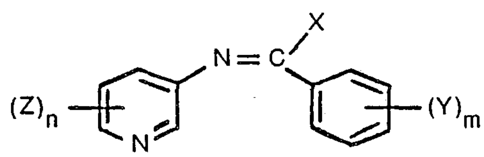

(I)

dans laquelle X est un alcoxy éventuellement substitué, un cycloalcoxy, un alcényloxy, un alcynyloxy, un aryloxy, un halogénoaryloxy, un alcoxyaryloxy, un aralkyloxy, un halogénoaralkyloxy, un alcoxy-aralkyloxy, un alkylthio éventuellement substitué, un cycloalkylthio, un alcénylthio, un alcynylthio, un arylthio, un halogénoarylthio, un alcoxyarylthio, un aralkylthio ou un groupe de formule:

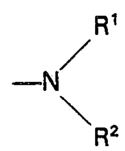

dans laquelle $R^1$ et $R^2$ sont choisis indépendamment parmi un hydrogène et un alkyle éventuellement substitué, ou $R^1$ et $R^2$ forment ensemble, avec l'atome d'azote intermédiaire, un noyau hétérocyclique de 5 à 7 atomes cycliques, un de ces atomes cycliques pouvant être un autre hétéroatome choisi parmi l'azote, l'oxygène et le soufre,

m est 0, 1 ou 2;

Y est un fragment alkyle, halogénoalkyle, alcoxy, halogénoalcoxy, halogéno, nitro, aryloxy, halogénoaryloxy, cyano ou alcoxycarbonyle;

n est 0, 1 ou 2; et

Z est un fragment alkyle, alcoxy ou halogéno.

2. Emploi comme revendiqué dans la revendication 1, dans lequel le dérivé est un composé de formule I, où X est un alcoxy, un alcoxyalcoxy, un alkylthio, un alkylamino ou un dialkylamino; m est 0, 1 ou 2; Y est un halogène; et n est 0.

3. Emploi comme revendiqué dans la revendication 1 ou 2, dans lequel le dérivé est un composé de formule I où X est un alcoxy en $C_{1-6}$, un alkylthio en $C_{1-6}$ ou un di-(alkyl en $C_{1-6}$)amino, Y est un halogène et n est 0.

4. Emploi comme revendiqué dans l'une quelconque des revendications 1 à 3, où le dérivé est un composé de formule I, où $(Y)_m$ est un substituant 4-halogéno.

5. Emploi comme revendiqué dans la revendication 4, où $(Y)_m$ est un substituant 4-chloro.

6. Emploi comme revendiqué dans la revendication 1, où le dérivé est un composé de formule I, où X est un cycloalcoxy, un aminoalkylamino ou un alcoxyalkylamino; m est 1 ou 2; Y est un halogène et n est 0.

7. Emploi comme revendiqué dans la revendication 6, où X est un chlore.

8. Emploi comme revendiqué dans l'une quelconque des revendications 1 à 3, 6 et 7, où le dérivé est un composé de formule I où Y est un chlore et m est 2, les substituants $(Y)_m$ étant dans les positions 2, 4 ou 3,4 du noyau benzène.

9. Emploi comme revendiqué dans la revendication 1, où le dérivé est un composé de formule I où X est un alcoxy, un aryloxy, un alkylthio, un monoalkylamino, un dialkylamino ou un noyau hétérocyclique azoté hexagonal fixé à l'atome de carbone par son atome d'azote; m est 0, 1 ou 2; Y est un halogène, un halogénoalkyle ou un cyano; et n est 0.

10. Une composition fongicide comprenant au moins un support inerte avec, comme ingrédient actif, au moins un dérivé de pyridyliminométhylbenzène à activité fongicide de formule I, ou un de ses

sels d'addition d'acides, comme défini dans l'une quelconque des revendications précédentes.

11. Un procédé pour préparer un composé de formule I, comme défini dans la revendication 1, qui comprend la réaction d'un composé de formule générale

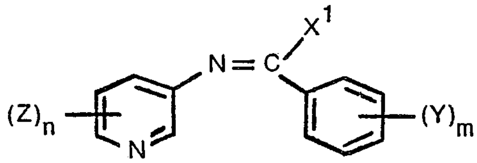

(II)

dans laquelle m, n Y et Z sont tous comme défini dans la revendication 1 et $X^1$ est un halogène, avec un composé de formule

Q—X

où X est comme défini dans la revendication 1, et Q est un àtome d'hydrogène ou un atome de métal alcalin, éventuellement en présence d'un accepteur d'acide.